# EUROPEAN PATENT APPLICATION

(11) **EP 4 454 483 A1**
(43) Date of publication of application: **30.10.2024**
(21) Application number: 22911128.1
(22) Date of filing: 16.12.2022
(51) Int. Cl.: A23L 33/10, A61K 31/341, A61P 25/00, A61P 25/28, C07D 307/33

(54) **BRAIN FUNCTION IMPROVING AGENT**

(30) Priority: 22.12.2021 JP 2021207910
(71) Applicant: OSAKA UNIVERSITY, Suita-shi Osaka 565-0871 (JP); MITSUI NORIN CO., LTD., Tokyo 105-8427 (JP)
(72) Inventor: AGO Yukio, Suita-shi, Osaka 565-0871 (JP); NAKAGAWA Shinsaku, Suita-shi, Osaka 565-0871 (JP); ISHIMOTO Kenji, Suita-shi, Osaka 565-0871 (JP); TAKAGAKI Akiko, Fujieda-shi, Shizuoka 426-0133 (JP); OKI Shogo, Fujieda-shi, Shizuoka 426-0133 (JP)
(74) Representative: Kordel, Mattias
(86) International application number: PCT/JP2022/046424
(87) International publication number: WO 2023/120425

(57) **Abstract**

The present invention pertains to a brain function improving agent for preventing the onset of, treating, and/or ameliorating cognitive function impairment and depressive symptoms in central nervous system diseases, the agent being characterized by containing as active ingredient, at least one among 5-(3,5-dihydroxyphenyl)-γ-valerolactone represented by formula (I), a salt of said compound, and a conjugate of said compound.

## Description

### Technical Field

The present invention relates to a brain function improving agent for preventing the onset of, treating, and/or ameliorating the symptoms of short-term memory impairment due to brain functional decline, and the cognitive impairment and depression due to the central nervous system diseases such as neurodegenerative disease and psychiatric disorder.

### Background Art

As people are aging, the prevalence rate of age-related central nervous system diseases such as dementia, neurodegenerative disease, and cerebrovascular disease rises sharply. The estimated number of the patients with dementia in the world is about 44 million and will triple in 2050. It is known that dementia is induced by the defect in the structure or the function of the nerve cells in the brain. The defect is caused by the change of the homeostasis-maintaining mechanism in the brain environment consisting of brain nerve and surrounding glial cells. Among the central nervous system diseases, the disease induced by the progressive neurological injuries is particularly referred to as neurodegenerative disease. Alzheimer's disease (AD) is a representative example of the disease. Other examples of the disease include Parkinson's disease (PD), Huntington's disease (HD), and amyotrophic lateral sclerosis (ALS).

It is reported that about 60 % of the Japanese patients with dementia are diagnosed as Alzheimer type dementia. Alzheimer type dementia is characterized by that forgetfulness is recognized from an early stage of the disease. The symptoms such as memory disorder which is referred to the inability to memorize new things and inability to remember, the lowering of the learning ability, the lowering of the judgement, and disorientation appear. Prevention and treatment of cognitive dysfunction caused by neurodegenerative disease including dementia described above are desired by many people, because the progressed symptoms pose a threat to live with human dignity when the symptoms progress.

Recently the number of the patients with psychiatric disorders such as depression and schizophrenia has tended to rise. In the survey conducted by the Ministry of Health, Labour and Welfare in Japan from fiscal 2013 to 2015, it was reported that the lifetime prevalence of depression was 5.7 %. According to the results of the survey of the patients conducted by the Ministry of Health, Labour and Welfare in Japan, it is reported that the total number of the patients with psychiatric disorder will be about 4.2 million in 2017 and that among them the number of the patients with depression is 1.27 million that is the highest patients in the patients with psychiatric disorder. When the psychiatric disorder such as depression develops, the physical symptoms such as sleeplessness, anorexia, and easy fatiguability appear with the psychiatric symptoms such as being depressed all day and finding no pleasure in doing anything, and the serious troubles occur in daily life.

Additionally, it is said that the number of the patients with schizophrenia which is one of the psychiatric disorders just like depression is about 800 thousand in Japan. The lifetime prevalence of schizophrenia is estimated to be 0.7 % of the total population in the world. In addition to hallucination and delusion, disordering of cognitive functions such as memorizing, thinking, understanding, calculating, learning, verbalizing and judging, are known to be main symptoms of schizophrenia. These symptoms cause troubles in the life and in the social activities in general. To improve social productivity, many companies are working on mental healthcare for preventing the onset of psychiatric disorder described above.

Both the neurodegenerative disease represented by dementia and the psychiatric disorders including depression and schizophrenia are thought to be central nervous system disease developed by the occurrence of the abnormality in the brain function. However, the underlying cause of those abnormal cerebral function is not identified clearly, and many of the diseases are intractable.

It is thought that the brain atrophy resulting from the decrease of the brain cells due to aging, the decrease of the neurotransmitter such as brain acetylcholine, and the like are involved in the cause of the onset of Alzheimer type dementia the number of the patient with which is largest among dementias. It is known that the patients with dementia have a significantly small amount of extracellularly acetylcholine in the brain, and many efforts to discover drugs by making the increase of acetylcholine outside the brain cells as an indicator (Non-Patent Document 1).

The triggers of the onset of psychiatric disorder such as depression and schizophrenia are an environmental factor, particularly, social and mental stress, as well as the genetic factor. Because inflammatory cytokine increases in the blood of the patients with depression and microglia being implicated in inflammation is activated in the brain of the patients with depression, the relevance of inflammation in the brain to depression is suggested. Recently, it has been shown that the stress loading induces an excessive inflammatory response in the glia cells, leading to sluggish response and atrophy in the brain nerve cells (Non-Patent Document 2).

In regard to the medicine for the disease with the abnormality in the brain function, currently there is no treatment and no medicine for effecting a radical cure of neurodegeneration that causes dementia. Many of the patients have a low satisfaction level with the medicine and also there are many side effect problems with the medicine for the brain disease. Donepezil, galatamine and rivastigmoine which slow down the progress of Alzheimer type dementia are the strong inhibitor for acetylcholine decomposing enzyme. These medicines have the side effects on the alimentary system such as nausea, vomiting, and diarrhea and other side effects such as bradycardia, which is thought to be problems. Memantine that is a NMDA receptor antagonist has side effects such as wobble, sleepiness, headache, and blood pressure elevation.

The antidepressant medicines referred to as tricyclic antidepressants and tetracyclic antidepressants have been used since before, recently SSRI, SNRI, and NaSSA referred to as new antidepressant are used as a representative antidepressant. The purpose of these medicines is to provide the antidepressant effect to enhance the information transmission in the brain by stimulating release of the extracellular neurotransmitter in the brain. The rate of the patients whose symptom is ameliorated by the treatment using the conventional antidepressant is 50 % and low. It is said that 30 % of the patients are the patients who suffer from treatment-resistant depression, that impair their QOL (Quality of Life) and rehabilitation into society.

Among central nervous system diseases, cognitive dysfunction of the aged is particularly hard to treat and has a high tendency to become worse gradually once onset occurs. It is thought that developing dementia at an advanced age is a main reason for needing the care and serious troubles occur in daily life. Thus, the great majority of the healthy persons hopes that the onset of dementia can be prevented, which is shown in the wide area questionnaire. With the social background described above, many people recognize that the lifestyle habit to prevent the onset of dementia and reexamining dietary habit are important.

In Japanese dietary habit the green tea is the drink that anyone regardless of age or gender likes in every situation. The health function of the ingredient of the green tea is widely and generally known. Particularly, there are many epidemiological studies are reported for the efficacies of antihypertensive, antihyperglycemic, and the like by the green tea catechin. Recently, the systematic review of the observation studies was conducted, in which the relevance of the intake of the green tea to the onset of dementia, Alzheimer's disease, mild cognitive impairment, and cognitive impairment were investigated (Non-Patent Document 3). The review indicated the possibility of drinking the green tea reduced the risk of dementia, Alzheimer's disease, mild cognitive impairment, or cognitive impairment.

Among the green tea catechin known as the functional ingredient of the green tea, epigallocatechingallate (EGCg) that is particularly largely contained is a compound having a high antioxidant activity and known to have various physiological activities. There are some study reports regarding the relevance of EGCg to cognitive function. It is reported that EGCg inhibit the generation of amyloid β in mouse model of the Alzheimer's disease. This is caused by the EGCg activation to the α-secretase pathway that reduce the production of amyloid β (Non-Patent Document 4). Additionally, the craniall nerve protecting action (Non-Patent Document 5) and the inflamatory cytokine reducing action on microglia (Non-Patent Document 6) by EGCg are reported.

While EGCg has been reported to be effective in improving cognitive function, but the amount absorbed into the body after ingestion (bioavailability) is extremely low (Non-Patent Document 7). Thus, recently there are some research focusing on the functionality of the EGCg metabolites produced by enteric bacterium, which are more highly absorbed in tha body than EGCg itself (Patent Documents 1 to 4).

It is reported that there are several compounds as EGCg metabolites produced by enteric bacterium. Among them, regarding 5-(3,5-dihydroxyphenyl)-γ-valerolactone, which is one of the main metabolites of the green tea catechin, and its conjugate, it is disclosed that the effect on the cranial nerve cell SH-SY5Y in vitro is investigated and that 5-(3,5-dihydroxyphenyl)-γ-valerolactone and its conjugate have a significant nerve cell proliferating action (Patent Document 5).

The compounds which have phenyl-γ-valerolactone as a basic skeleton are reported to be the metabolites produced by enteric bacterium from the compounds having a flavan-3-ol skeleton such as catechin and procyanidin (Non-Patent Document 8). As an example of the study reports about an action of phenyl-γ-valerolactone on the cognitive function, it is reported that 5-(4-monohydroxyphenyl)-γ-valerolactone reduces the cytotoxicity induced by β-oligomer (Non-Patent Document 9). However, it is only reported that when 5-(4-monohydroxyphenyl)-γ-valerolactone which is pre-incubated with amyloid β is directly injected into the cerebral ventricles of mouse, there is no mouse with impairment in the mouse. Which means that, the efficacy of 5-(4-monohydroxyphenyl)-γ-valerolactone on the brain function in the living body, when 5-(4-monohydroxyphenyl)-γ-valerolactone is administrated, are completely unknown.

### Citation List

### Patent Document

Patent Document 1: JP 2012-144532 A
Patent Document 2: JP 2015-030724 A
Patent Document 3: JP 2016-003200 A
Patent Document 4: JP 2016-160238 A
Patent Document 5: JP 2017-061438 A

Non-Patent Document 1: Annual Review of Medicine, 2006, Vol.57, p.513-533
Non-Patent Document 2: Neuron. 2018 Aug 8; 99(3): 464-479
Non-Patent Document 3: Nutrients, 2019, Vol.11(5), 1165. doi: 10.3390/nu11051165
Non-Patent Document 4: Neurosci. 2005, Vol.25, p.8807-8714. [16177050]
Non-Patent Document 5: Neurosci Lett. 2000. Vol.287, p.191-194. [10863027]
Non-Patent Document 6: The Journal of Nutritional Biochemistry,2014, Vol.25(7), p.716-725
Non-Patent Document 7: Journal of Agricultural and Food Chemistry, 2001, Vol.49(8), p.4102-4112.
Non-Patent Document 8: Natural Product Reports, 2019, Vol.36, p.714-752
Non-Patent Document 9: Molecular Nutrition & Food Research, 2020, Vol.64(5), 1900890,doi.org/10.1002/mnfr.201900890

### Summary of Invention

### Technical Problem

Regarding cognitive dysfunction and depressive symptoms of the central nervous system diseases such as dementia, depression, and schizophrenia, the onset and the mechanism conditions are hardly clarified and the effective treatment is not well-established. It is reported that many patients with central nervous system diseases have low satisfaction with their medicine, with only about 50% patients of those treated with antidepressants are realizing improvement of their symptoms, thus there are limitations to medication. Regarding to dementia, the existing therapeutic agent, the acetylcholine-degrading enzyme inhibitor has side effects such as nausea, vomiting and bradycardia, thus it is difficult to effect a radical cure by medication. Central nervous system diseases such as dementia, depression and schizophrenia are difficult to cure once they have developed, and thus interfere with social life and reduce QOL (Quality of Life). Therefore, the importance of prevention through self-medication and maintaining an unaffected state is recognized.

There are some commercial daily supplements to prevent the onset of dementia, depression and the like. Omega-3 fatty acid is known as a representative material. The market for the supplement of omega-3 fatty acid including DHA and EPA is large and awareness in general of the supplement is high. However, a study of clinical trial to test the efficacy of omega-3 fatty acid supplements for the depression mood that it was unclear whether omega-3 fatty acid supplements were useful for the general older population. It is reported in the review investigated clinical trials that it was no evidence for the benefit from the omega-3 fatty acid supplement in Alzheimer's dementia.

Besides, the supplement containing ginkgo leaf extract as an active ingredient is generally well-known but it is contraindicated in patients taking anticoagulant. Thus, there is a problem that it is not fit for the middle-aged and the aged. As described above, several supplements for maintaining and ameliorating cognitive function are already on the market. But there are products having a restriction in their use and there are few products by which excellent effect is obtained. Regarding depressive symptoms the supplement of St. Jones Wert (herb) is on the market. But because there are risks of overdose and a plurality of risk information of the interaction with medicines, there are some problems with daily intake. As described above, the supplements for cognitive function and depressive symptoms have problems due to their active ingredients, and furthermore, there are few reports of supplement efficacy on humans.

On the other hand, it is known that 5-(3,5-dihydroxyphenyl)-γ-valerolactone as one of the green tea metabolites produced by enteric bacteria and the conjugate have a significant action of proliferating the cerebral nerve cell SH-SY5Y (Patent Document 5). However, it means that only the enhancement effect of cell prolifertive effect was confirmed, but it was not confirmed what symptoms of brain dysfunction in an actual living body were improved. The phenomenon of enhanced the cerebral nerve cell SH-SY5Y proliferation cannot say that the agent is effective or immediate when administered orally in an actual living body.

In view of the above, the purpose of the present invention is to provide a brain function improving agent containing the active ingredient derived from food. The targets of this agent are short-term memory impairment due to the brain functional decline of the central nervous system, and the symptoms of cognitive dysfunction and depression in the dementia, depression and schizophrenia which are diseases of the central nervous system. And this agent can be used for improving these symptoms, preventing the onset of these diseases or treating.

### Solution to Problem

By the earnest research of the improving action of cognitive function and depressive symptoms in a living body by the ingredient derived from food, the present inventors found that the amount of extracellularly acetylcholine that is a neurotransmitter is significantly increased in the mouse cerebral cortex by orally administering 5-(3,5-dihydroxyphenyl)-γ-valerolactone which is one of the metabolite from catechin. When multiple behavior tests were conducted by using a mouse to check the effectiveness of the compound in a living body, the short-term working memory was improved significantly in the amnesia mouse which scopolamine was administered to. Depressive symptoms were confirmed to be ameliorated in the experiments using mouse with brain inflammation induced by the administration of LPS (Lipopolysaccharide) and the mouse reared in long-term social isolation. In addition, experiments with long-term socially isolated mouse showed not only an improvement in the symptoms of cognitive dysfunction, but also a suppression of the onset of dysfunction. The onset of cognitive dysfunction was confirmed to be prevented by long-term administration of the compound. From these results, the present inventors have found that 5-(3,5-dihydroxyphenyl)-γ-valerolactone provides an excellent brain function improving agent having the effective ameliorating action of cognitive dysfunction and depressive symptoms in a living body and the inhibiting action of the decline in cognitive function and the onset of depressive symptoms so as to complete the present invention.

The present invention provides a new agent for improving, preventing the onset of, or treating the symptoms of short-term memory impairment caused by brain functional decline in the central nervous system, and cognitive dysfunction and depressive symptoms in the central nervous system diseases such as dementia, depression, and schizophrenia, and whose active ingredients are 5-(3,5-dihydroxyphenyl)-γ-valerolactone, its salt, and its conjugate. Because the active ingredient of the present invention is derived from food, the present invention can be widely applied to the supplement, the food and drink, the medicine, and the like, is safe and has high versatility. The present invention can lead to the prevention and/or the amelioration of cognitive dysfunction and depressive symptoms.

5-(3,5-dihydroxyphenyl)-γ-valerolactone is reported as the metabolite produced by enteric bacterium from flavan-3-ols represented by green tea catechin. The green tea has the history of drinking everyday over the years and the safety of the metabolite of the green tea catechin produced in the intestine can be said to be confirmed empirically.

That is, the present invention relates to:
[1] A brain function improving agent for preventing onset of, treating, and/or ameliorating central nervous diseases, characterized by containing, as an active ingredient, at least one of 5-(3,5-dihydroxyphenyl)-γ-valerolactone represented by a following formula (I): 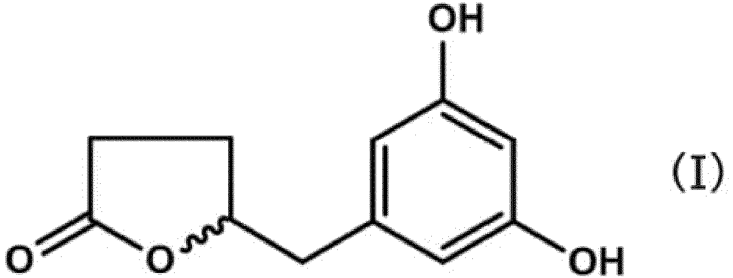
   (In the formula, a wavy line represents that the steric configuration is R-configuration or S-configuration.),
   a salt of said compound, and a conjugate of said compound.
[2] The brain function improving agent according to [1], wherein the central nervous diseases are neurodegenerative diseases or psychiatric disorder.
[3] The brain function improving agent according to [1] or [2], wherein central nervous diseases are diseases accompanied by cognitive dysfunction.
[4] The brain function improving agent according to any one of [1] to [3] that activates muscarinic receptors by enhancing acetylcholine release and increasing extracellular acetylcholine in the brain.
[5] A composition for preventing onset of, treating, and/or ameliorating central nervous diseases, containing the brain function improving agent according to any one of [1] to [4].
[6] A supplement for improving brain function containing the composition according to [5].
[7] Food and drink for improving brain function containing the composition according to [5].
[8] Medicine or quasi-drugs for improving brain function containing the composition according to [5].
[9] An agent for enhancing acetylcholine release or increasing extracellular acetylcholine in the brain characterized by containing, as an active ingredient, at least one of 5-(3,5-dihydroxyphenyl)-γ-valerolactone represented by the following formula (I): 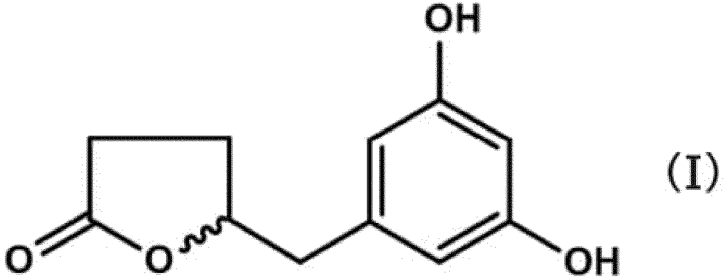
   (In the formula, wavy line represents that the steric configuration is R-configuration or S-configuration.),
   a salt of said compound, and a conjugate of said compound.

### Effects of the Invention

One of the present inventions relates to the brain function improving agent for preventing the onset of, treating, and/or ameliorating the symptoms of short-term memory impairment due to brain functional decline, and the cognitive impairment and depression due to the central nervous system diseases such as neurodegenerative disease and psychiatric disorder, and characterized by containing, as an active ingredient, at least one among 5-(3,5-dihydroxyphenyl)-γ-valerolactone, a salt of said compound, and a conjugate of said compound. Because of containing the ingredient derived from food as the active ingredient, the brain function improving agent of the present invention has high safety and immediate effects. The present invention is safe, has high versatility and can be widely applied to the supplement, the food and drink, the medicine, and the like. By using the present invention, the functional food, the supplement, and the medicine having high safety, a safe and excellent preventing action of the onset of, and an safe and excellent ameliorating action of cognitive dysfunction and depressive symptoms can be provided.

### Brief Description of Drawings

[Fig.1] Fig.1 shows the change in the amount of the extracellular acetylcholine in the cerebral cortex of the ddY mouse in Test Example 1.
[Fig.2] Fig.2 shows the spontaneous alternation behavior rate of the amnesic mouse by scopolamine when 5-(3,5-dihydroxyphenyl)-γ-valerolactone (compound A) is administered orally in Test Example 2.
[Fig.3] Fig.3 shows the exploratory time and discrimination index of the model mouse developing cognitive dysfunction by long-term isolated rearing when orally administering different amount of 5-(3,5-dihydroxyphenyl)-γ-valerolactone (compound A) to the model mouse in Test Example 3.
[Fig.4] Fig.4 shows the exploratory time and discrimination index of the mouse when they were kept isolation and continuously fed the MF powdery diet containing 5-(3,5-dihydroxyphenyl)-γ-valerolactone (compound A) at a rate of 7 mg/100 g for 5 weeks in Test Example 4.
[Fig.5] Fig.5 shows the immobility time of the mouse orally administrated with 5-(3,5-dihydroxyphenyl)-γ-valerolactone (compound A) during the test when administrating LPS which caused inflammation in brain in Test Example 5.
[Fig.6] Fig.6 shows the immobility time of the mouse reared in long-term isolation during the test when administrating 5-(3,5-dihydroxyphenyl)-γ-valerolactone (compound A) orally in Test Example 6.

### Form to carry out invention

The present invention relates to a brain function improving agent for preventing the onset of, treating, and/or ameliorating the symptoms of short-term memory impairment due to brain functional decline, and the cognitive impairment and depression due to the central nervous system diseases such as neurodegenerative disease and psychiatric disorder, and characterized by containing, as an active ingredient, at least one among 5-(3,5-dihydroxyphenyl)-γ-valerolactone, a salt of said compound, and a conjugate of said compound.

Examples of central nervous diseases in the present invention include the symptoms such as Alzheimer type dementia, Parkinson's disease, Huntington's disease, senile dementia, dementia with Lewy bodies and the like caused by neurodegenerative disease and psychiatric diseases such as depression, manic-depressive psychosis, schizophrenia, autism spectrum disorder, anxiety disorder, and the like caused by brain dysfunction.

The effects of preventing the onset of, treating, and/or ameliorating in central nervous diseases in the present invention are based on the mechanism of action that the muscarinic receptor is activated by enhancing the release of acetylcholine and increasing the amount of extracellular acetylcholine in the cerebral cortex by the action of 5-(3,5-dihydroxyphenyl)-γ-valerolactone, a salt of the compound, and a conjugate of the compound. The choline hypothesis in which the onset of Alzheimer type dementia is mainly caused by the decline in the function of cholinergic nerve in the brain was proposed by Whitehouse et al in 1982. Muscarinic receptor which is one of the acetylcholine receptor binding acetylcholine is divided into five subtypes which are all membrane protein referred to as G protein-coupled receptor (GPCR) and is known to be the drug discovery target. It is reported that the recall of the memory was impaired in the amnesia model rat administered with scopolamine which is the muscarinic acetylcholine receptor antagonist (Front. Aging Neurosci., 08 April 2014, doi. org/10.3389/fnagi. 2014. 00063) and the model rat is used as representative evaluation system for the cognitive function.

Neurodegenerative diseases and psychiatric disorders in central nervous system diseases are caused by long-term psychiatric stress or brain inflammation, and by taking the compound represented by formula (I) of the present invention, psychiatric disorders due to long-term stress or brain inflammation are ameliorated.

The brain function improving agent of the present invention ameliorates the symptoms of central nervous system diseases causing memory disorder. Memory is said to have mainly three functions of memorization (memorizing), keeping (keeping the things memorized), and recall (recalling the information memorized). Sensory memory (momentary memory), short-term memory (the memory kept in mind for a comparatively short time), and long-term memory (the memory kept for a comparatively long time) are defined as three kinds of memories. The brain function improving agent of the present invention can ameliorate disorder of these memories. There are several behavior test systems of animal for evaluating these memories. Y-maze test, water-finding test, novel object recognition test, passive avoidance test, and the like are known as a representative evaluation system for memory and learning ability using mouse. Y-maze test is known as the test system for evaluating spatial working memory and short-term working memory kept through the activity coordination between prefrontal area and hippocampus. Novel object recognition test is a test using the Rodentia's characteristics of having a preference for the novelty and known as the memory learning test targeting "the shape or the position of the object" and the test system for evaluating the visual perception memory. These are effectively evaluated using the amnesia model animal administered with scopolamine which is the muscarinic acetylcholine receptor antagonist. For example, the efficacy of ameliorating spatial working memory and short-term working memory can be judged by Y-maze test using the amnesia model mouse by the administration of scopolamine hydrochloride.

The brain function improving agent of the present invention ameliorates the symptoms of psychiatric disorder in central nervous system diseases. Examples of psychiatric disorder include depression, manic-depressive psychosis, schizophrenia, autism spectrum disorder, and anxiety disorder and the psychiatric disorder means the state in which there is a bias in the emotion and the behavior due to the disorder and the injury of the brain and the other cause. These psychosis disorder can be induced by isolating and rearing the experimental animals for a long time. By conducting the novel object recognition test using the psychosis disorder model mouse, the effect of alleviating the symptoms of, the therapeutic effect of and the effect of inhibiting the onset of cognitive dysfunction (specifically memory learning ability or visual perception memory to memorize the shape or the position of the object) which are the symptoms of psychiatric disorder can be evaluated.

Regarding the onset mechanism of psychiatric disorder such as depression, the involvement of several substances in the body such as the deficiency of monoamine in the brain and the decrease in brain-derived neurotrophic factor is reported. Recently it is reported that the inflammatory cytokine secreted from the microglia cell by the stress cause inflammation in the brain to lead the onset of depression as one of the onset mechanisms of psychiatric disorder including depression and schizophrenia. LPS (Lipopolysaccharide) is glycolipid that forms Gram-negative bacteria cell wall outer membrane and is known to enhance the secretion of various inflammatory cytokine through signal transformation pathways. It is reported that LPS causes the decrease in brain-derived neurotrophic factor and the variation of the density of dendrite of neuron and that accordingly depression-like behavior is caused. Because the brain function improving agent of the present invention have ameliorating action of depressive symptoms on the model mouse with depressive symptoms induced by LPS, it can inhibit the onset of depressive symptoms caused by inflammation in the brain.

By administering the brain function improving agent of the present invention into the body, the amount of free acetylcholine is increased in the brain (cerebral cortex). Thus 5-(3,5-dihydroxyphenyl)-γ-valerolactone represented by the formula (I) which is the active ingredient can be acetylcholine release-enhancing agent or increasing extracellular acetylcholine. The increasing action of the amount of free acetylcholine has immediacy and the amount of free acetylcholine in the brain is increased rapidly after the administration. The amount of free acetylcholine in the brain become maximum in 20 to 60 minutes after the administration and the state in which the amount is increased is maintained until 100 minutes after the administration. The effect of the present invention of preventing the onset of, the treating, and/or the ameliorating in central nervous diseases is based on the action mechanism that muscarinic receptor is activated by enhancing the release of the extracellular acetylcholine in cerebral cortex, which can be confirmed by evaluating the effect by combining the measurement of the amount of free acetylcholine in the brain and the behavior tests regarding short-term working memory and the like such as Y-maze test. It is known that Donepezil hydrochloride (commercial name Aricept), galantamine (commercial name REMINYL), rivastigmine (commercial name EXELON or RIVASTACH patch) which are generally prescribed as a drug for treatment of Alzheimer type dementia and the like inhibit acetylcholine esterase which is hydrolase of acetylcholine reversibly to suppress the degradation of acetylcholine and that as a result, the concentration of acetylcholine is increased at the site of action (in the brain) to enhance neural transmission of cholinergic nerve. Because it is confirmed that the compound represented by the formula (I) of the present invention do not provide inhibiting action of acetylcholine esterase (the date is not described), its action mechanism is different from that of acetylcholine enhancing agent described above.

The compound which is the active ingredient of the brain function improving agent of the present invention is 5-(3,5-dihydroxyphenyl)-γ-valerolactone represented by the formula (I).

The compound represented by formula (I) is described. 5-(3,5-dihydroxyphenyl)-γ-valerolactone represented by the formula (I) is as follows.

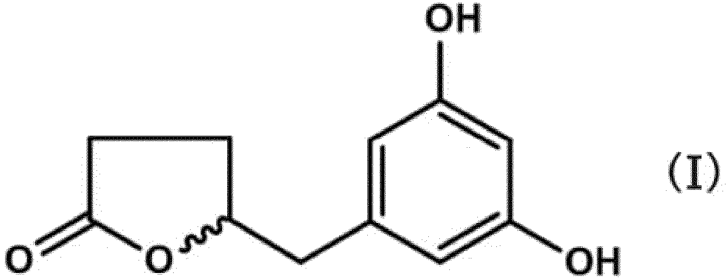

In the formula (I), the wave line represents that the configuration is either of R configuration or S configuration. Thus, the compound represented by the formula (I) includes both of R form and S form. Note that R form is preferable. The compound represented by the formula (I) is known to be the metabolite produced from tea catechins in the intestine when taking epigallocatechin or epigallocatechin gallate orally. The manufacturing method of the compound is well-known. For example, Patent Document JP2011-87486 A discloses the production method in which the compound is produced by microorganism from epigallocatechin or the production method in which the compound is produced in the presence of the cultured microorganism preparation. In addition, the compound can be obtained by the well-known method for organic chemical synthesis (synthesis, 9, 1512-1520, 2010) and the like.

5-(3,5-dihydroxyphenyl)-γ-valerolactone is the metabolite produced by entric bacterium from epigallocatechin, epigallo catechin gallate, gallocatechin, or gallocatechin gallate. Actually, it is reported that the sulfate conjugate and the glucuronic acid conjugate of the compound of 5-(3,5-dihydroxyphenyl)-γ-valerolactone are detected in the serum of the mouse when orally administering 5-(3,5-dihydroxyphenyl)-γ-valerolactone to the mouse (Biol. Pharm. Bull, 42, 212-221, 2019). Accordingly, it is well-known that the conjugates are presence in the body of the mouse after orally administering 5-(3,5-dihydroxyphenyl)-γ-valerolactone, which suggests the possibility that the conjugates contribute the biofunctional property after orally administering 5-(3,5-dihydroxyphenyl)-γ-valerolactone. Hence, the ameliorating action of cognitive function in the living body described in this specification can be regarded to include the effect of the conjugates of 5-(3,5-dihydroxyphenyl)-γ-valerolactone such as the sulfate conjugate and/or the glucuronic acid conjugate.

Namely, it is considered that the conjugate of 5-(3,5-dihydroxyphenyl)-γ-valerolactone as well as the compound represented by the formula (I) which is the active ingredient having brain function improving agent of the present invention also have brain function improving effect.

The conjugate of the compound represented by the formula (I) is described in detail further. Examples of the conjugate include the sulfate conjugate and/or the glucuronic acid conjugate. The sulfate conjugate of 5-(3,5-dihydroxyphenyl)-γ-valerolactone is the compound obtained by replacing at least one hydroxy group in the compound represented by the formula (I) with a sulfate group or a salt thereof. The sulfate group or the salt thereof is either of the substituents described below.

-OSO₃H

-OSO₃⁻

Example of the compound containing at least one sulfate group or the salt thereof as a substituent can be obtained by sulfonating at least one hydroxy group. The sulfonation can be carried out by using sulfur trioxide pyridine complex and the like.

The glucuronic acid conjugate of 5-(3,5-dihydroxyphenyl)-γ-valerolactone is a compound which is obtained by replacing at least one hydroxy group in the compound represented by the formula (I) with the substituent containing glucuronic acid or the salt of glucuronic acid. The substituent containing glucuronic acid or the salt of glucuronic acid is either of the substituents described below.

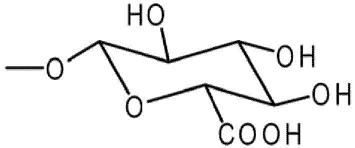

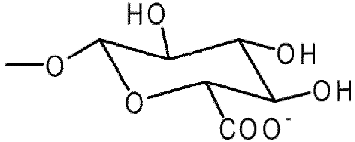

Examples of the compound obtained by replacing a hydroxy group in the compound represented by the formula (I) with glucuronic acid or the salt of glucuronic acid include the metabolite of the tea catechins which is detected in urine, when orally taking epigallocatechin and the like. Examples of the methods for manufacturing the conjugate include the method in which the conjugate in the urine collected from the rat administered with epigallocatechin is separated and collected by using HPLC and the like.

As described above, the compounds contained in the present invention can be obtained from epigallocatechin or is the metabolite of epigallocatechin, epigallocatechin gallate, or gallocatechin, gallocatechin gallate or the derivative of the metabolite. Catechins [epigallocatechin ((-)-epigallocatechin), epigallocatechin gallate ((-)-epigallocatechin gallate), gallocatechin ((-)-gallocatechin), gallocatechin ((-)-gallategallocatechin gallate)] are mainly contained in the green tea and obtained by extracting the leaves, the stem, the xylem, the bark, the root, the fruit, the seed of the tea and the mixture of them or the pulverized product of them with water, hot water, organic solvent, water-containing organic solvent or the mixture of them. catechins is obtained by extracting the raw tea leaves or the dried tea leaves with, water, hot water, organic solvent, water-containing organic solvent or the mixture of them. Besides the extract itself there is the purer form of the extract and the like. The form of catechins may be either of liquid or solid (including power).

The brain function improving agent of the present invention can contain the salt of the compound represented by the formula (I) which have similar brain function improving effect as the active ingredient. The salt of the compound represented by the formula (I) is preferably the pharmacologically permissible salt. Examples of these salt include alkali metal salt such as sodium salt and potassium salt, alkaline earth metal salt such as calcium salt and magnesium salt, ammonium salt, and organic base such as methylamine, ethylamine, butylamine, dimethylamine, diethylamine, triethylamine, tributylamine, ethanolamine, pylidine, lidine, and arginine.

These salts can be obtained by the conventional method, for example, sodium salt can be obtained by bringing the compound represented by the formula (I) into contact with sodium hydroxide.

The composition of the present invention for preventing the onset of, treating, and/or ameliorating central nervous diseases (hereinafter also referred to as the composition for improving brain function) contain the compound represented by the above formula (I). When administering the compound to the human body, the compound is administered so as not to adversely affect the human body. In the present invention the administering amount of the compound represented by the formula (I) to human may be determined depending on weight, sex, age, or other factors appropriately. For example, when administering orally, the administering amount is preferably 10 to 500 mg per day per adult human who weighs 60 kg, more preferably 30 to 300 mg, further preferably 50 to 150 mg. For example, the compound in an amount described above is preferably divided in 1 to 3 times per day to administer.

Examples of the embodiment of the present invention include the composition, the medicine and quasi-drug, the food and drink, the supplement containing the compound represented by the formula (I). When the embodiment is the medicine, the dosage form can be tablet, capsule, injection, infusion liquid, powder, suppository, granule, ointment, suspension, emulsion, syrup, cream and the like depending on the purpose and the route of the administration.

The medicine can contain the additives which are generally used for formulation such as the binder, the excipient, the lubricant, the disintegrator, the stabilizer, the emulsifier, the buffer. The preferable examples of the binder include guar gum and gum arabic powder. The preferable examples of the excipient include starch, trehalose, and dextrin. The preferable examples of the lubricant include stearic acid, talc, sucrose fatty acid ester, polyethylene glycol. The preferable examples of the disintegrator include starch, carboxymethylcellulose and cornstarch. The preferable examples of the stabilizer include oils and fats, propylene glycol, cyclodextrin. The preferable examples of the emulsifier include anionic surfactant, nonionic surfactant, polyvinylalcohol. The preferable examples of the buffer include the buffer solution of phosphate, carbonate, citrate, and the like.

The compound represented by the formula (I) can be contained in the food and drink. The food and drink contain the compound so as not to adversely affect the human body when eaten or drunken. In addition, by containing the compound represented by the formula (I), the food and drink can become the functional food aimed at the effect of maintaining, enhancing, and improving cognitive function. When the improving agent of the present invention is used as the food and drink or the functional food, examples include the composition containing 0.01 to 10 % by mass of the compound represented by the formula (I).

The kinds of the food and drink in which the compound contained include the drinks such as the juice, the soft drink, the tea, the processed foods such as the bread and the rice cake, the snacks and confectionery such as the candies, the instant foods such as the instant noodles, the oils and fats such as the butter and the salad oil, the condiments such as the dressing, the mayonnaise, the worcestershire sauce, the soy sauce, or the mirin (sweet sake), the furikake (rice seasoning)and the miso (soybean paste) and the compound can be contained in the wide-ranging food and drink. The supplement prepared by using the excipient appropriately can also be provided.

### Examples

The present invention is now described in more detail with reference to Examples as follows and is not limit to these Examples only for describing the present invention preferably.

### <Manufacturing Example 1: Manufacture of (R)-5-(3,5-dihydroxyphenyl)-γ-valerolactone>

Eggerthella lenta strain JCM9979 was inoculated into 30 ml of GAM broth media and was incubated under the anaerobic conditions at 37 °C for 48 hours to obtain the preculture solution. Escherichia coli strain K12 and Eubacterium plautii (Flavoifractor plautii) strain ATCC29863 were incubated in 10 ml of GAM broth media under the anaerobic conditions for 24 hours to obtain the preculture solutions. The preculture solutions of strain JCM9979 and Escherichia coli strain K12 were added to 100 ml of GAM broth media containing 290 mg of (-)-epigallocatechin and incubated under the anaerobic condition at 37 °C for 48 hours. 1 ml of sample was taken from the culture solution and centrifuged at high-speed (15000×g, 10 minutes) to remove the bacteria cell. The supernatant was analyzed by LC/MS to confirm the production of (S)-1-(3,5-dihydroxyphenyl)-3-(2,4,6-trihydroxyphenyl)-propane-2-ol. The conditions of the LC/MS are described below.

### <Conditions of LC/MS>

- Column: CAPCELLPAK C18 MG (2.0i.d. × 100.0 mm, 5 um, manufactured by Shiseido Co., Ltd.)
- Flow Velocity: 0.2 ml/min.
- Temperature of Column: 40 °C
- Mobile Phase:
   Solvent A: (water/acetonitrile/acetic acid = 100:2.5:0.1, volume ratio (v/v/v))
   Solvent B: (water/acetonitrile/methanol/acetic acid = 35:2.5:65:0.1, volume ratio (v/v/v/v))
   Gradient; 0 min.: A 100 % B 0 %, 3 min.: A 100 % B 0 %, 25 min.: A 0 % B 100 %, 25.1 min.: A 100 % B 0 %, 33 min.: A 100 % B 0 %
- Detector: UV 270 nm
- Interface: ESI
- Polarity: negative

Next, the preculture solution of strain ATCC29863 described above was added to the culture solution described above in which the production of (S)-1-(3,5-dihydroxyphenyl)-3-(2,4,6-trihydroxyphenyl)-propane-2-ol was confirmed and incubated under the anaerobic conditions at 37 °C for 48 hours. After that, 1 ml of sample was taken from the culture solution and centrifuged at high-speed (15000×g, 10 min.). The supernatant obtained was analyzed by LC/MS described above to confirm the production of (R)-5-(3,5-dihydroxyphenyl)-γ-valerolactone (compound A).

The culture solution in which the production of (R)-5-(3,5-dihydroxyphenyl)-γ-valerolactone was centrifuged at high-speed (10000×g, 20 min., 10 °C) to remove the bacteria cell. Hydrochloric acid was added to the supernatant to adjust the pH to be 3.5, then the solution was extracted with 200 ml of ethyl acetate 3 times. The ethyl acetate layer was concentrated and dried by evaporator and then subjected to the preparative HPLC. The conditions of the preparative HPLC are described below.

### <Conditions of Preparative HPLC>

·Column: CAPCELL PAK MG (20i.d.×150 mm, 5 um, manufactured by Shiseido Co., Ltd.)
·Flow Velocity: 15 ml/min.
·Temperature of Column: 40 °C
·Mobile Phase:
   Solvent A: acetonitrile:methanol:water:acetic acid (5:5:90:0.3, volume ratio (v/v/v/v))
   Solvent B: acetonitrile:methanol:water:acetic acid (5:65:30:0.5, volume ratio (v/v/v/v))
   Gradient; 0 min.: A 80 % B 20 %, 5 min.: A 80 % B 20 %, 20 min.: A 10 % B 90 %, 25 min.: A 10 % B 90 0,26 min.: A 80 % B 20 %, 35 min.:A 80 % B 20 %
·Detector: UV 270 nm

After fractionating and collecting, the analysis was conducted under the same condition as LC/MS analysis described above to identify the fraction containing the desired metabolites. Then the fraction was concentrated and dried by evaporator. 5 ml of pure water was added to dried and solid substance to dissolve and the solution was concentrated and dried under reduced pressure again. By repeating this operation 3 times the acid contained in the organic solvent was completely removed. A little of pure water was added to the dried and solid substance to dissolve and the solution was freeze-dried. 45.0 mg of (R)-5-(3,5-dihydroxyphenyl)-γ-valerolactone (compound A) was obtained.

### <Manufacturing Example 2: Manufacture of (R)-5-(3-sulfoxy-5-hydroxyphenyl)-γ-valerolactone (Compound of Present Invention Which is Sulfate Conjugate (Containing Sulfate Group) of Compound Represented by Formula (I))>

10 ml of pyridine and 5 mg of sodium sulfate were added to 163.1 mg (0.78 mmol) of (R)-5-(3,5-dihydroxyphenyl)-γ-valerolactone obtained in Manufacturing Example 1 and stirred at the room temperature for 10 minutes. Next, 790.4 mg (4.68 mmol) of pyridine-sulfur trioxide complex was added and stirred at the room temperature for 1 hour. After stirring, the reaction was stopped by adding 0.2 M sodium dihydrogen phosphate buffer (pH 7.2). The concentrated residue obtained by concentrating under reduced pressure by evaporator was dissolved in 3 ml of water and subjected to the preparative HPLC. The condition of the preparative HPLC was described below.

### <Conditions of Preparative HPLC>

·Column: CAPCELLPAK MG (20i.d.×150 mm, 5 um, manufactured by Shiseido Co., Ltd.)
·Flow Velocity: 19.0 ml/min.
·Temperature of Column: 40 °C
·Mobile Phase:
   Solvent A: sodium perchlorate/water (122.4:500, weight/volume ratio (w/v))
   Solvent B: sodium perchlorate/acetonitrile (122.4:500, weight/volume ratio (w/v))
   Gradient; 0 min.: A 100 % B 0 %, 3 min.: A 100 % B 0 %, 15 min.: A 0 % B 100 %, 16 min.: A 100 % B 0 0,20 min.: A 100 % B 0 %
·Detector: UV 280 nm

Acetonitrile was removed by concentrating the fraction under reduced pressure by evaporator to obtain the roughly purified (R)-5-(3-sulfoxy-5-hydroxyphenyl)-γ-valerolactone. Each roughly purified product obtained was introduced into the column obtained by additionally filling SUPELCO DISCOVERY DSC-18 column with ODS (Chromatorex 15-30 µm, 10 cm³). After washing with 160 ml of water, the roughly purified product was eluted with 175 ml of acetonitrile. The acetonitrile elution fractions obtained were concentrated under reduced pressure by evaporator and freeze-dried respectively to obtain 12.5 mg (0.04 mmol, yield 5.2 %) of purified (R)-5-(3-sulfoxy-5-hydroxyphenyl)-γ-valerolactone in white powder form.

The chemical shift value described below was obtained by 1H-NMR analysis of the purified product obtained and it was confirmed that the purified product obtained was the desired compound.

(R)-5-(3-sulfoxy-5-hydroxyphenyl)-γ-valerolactone: 1H-NMR (400MHz, methanol deuteride): δ6.24 (2H, s), 6.19 (1H, s), 4.79 (1H, m), 2.91 (2H, d, j=6.8Hz), 2.55 (1H, m),2.46(1H, m), 2.33 (1H, m), 2.00 (1H, m)

### <Manufacturing Example 3: Manufacture of (R)-5-[3-(β-D-gulcopyranuronosyloxy-5-hydroxy) phenyl]-γ-valerolactone (Compound of Present Invention Which is Glucuronic acid Conjugate (Containing Glucuronic Acid Group) of Compound Represented by Formula (I))>

Six 8-weeks-old Wistar series rats (male, purchased from Charles River Laboratories Japan, Inc.) preliminarily reared on the purified feed for a week were fasted from the day before the test and used for the test. The sample for administering was the solution obtained by dissolving (-)-epigallocatechin in the physiological saline solution. 20 mg/1.7 ml of the sample were forcibly administered to each rat into the stomach. The administration was repeated for 4 days. The urine was collected for 6 to 24 hours after the administration respectively.

250 ml of the urine collected were put together and concentrated by evaporator under the reduced pressure to reduce the volume of the urine to about 45 ml. Next, 225 ml of methanol were added to the concentrate obtained and stirred and then centrifuged at high speed (10000×g, for 10 minutes, 4 °C) to remove the precipitated protein. The supernatant obtained was concentrated by evaporator under the reduced pressure to reduce the volume to about 45 ml. After water was added to the concentrate obtained to increase the volume to 450 ml, pH was adjusted to be 3. 0 by adding acetic acid. After the adjusted urine solution was passed through OasisHLB cartridge (manufactured by Waters Corporation, 35 cc) whose pH was adjusted to be 3.0 with 0.1 M phosphate-citrate acid buffer, the cartridge was washed with 175 ml of water and 175 ml of 20 % methanol aqueous solution in this order. Then the fraction containing (R)-5-[3-(β-D-gulcopyranuronosyloxy-5-hydroxy) phenyl]-γ-valerolactone was eluted with 175 ml 40 % methanol aqueous solution. The 40 % methanol elution fraction obtained was concentrated by evaporator under reduced pressure and 5 ml of water were added to the concentrated residue and the solution was subjected to the preparative HPLC. The conditions of the preparative HPLC are described below.

### <Conditions of Preparative HPLC>

·Column: Mightysil RP18 GP (20i.d.×250 mm, 5 um, manufactured by KANTOU CHEMICAL CO., INC.)
·Flow Velocity: 15 ml/min.
·Mobile Phase:
   Solvent A: methanol/water/acetic acid (5/95/0.2, volume ratio (v/v/v))
   Solvent B: methanol/water/acetic acid (60/40/0.2, volume ratio (v/v/v))
   Gradient; 0 min.: A 80 % B 20 %, 5 min.: A 80 % B 20 %, 15 min.: A 0 % B 100 %, 18 min.: A 0 % B 100 %, 18.1 min.: A 80 % B 20 %, 25 min.:A 80 % B 20 %
·Detector: UV 270 nm

After fractionation and collection, the fractionated solution was subjected to LC/MS analysis (under the same conditions as in Manufacturing Example 1) to confirm that (R)-5-[3-(β-D-gulcopyranuronosyloxy-5-hydroxy) phenyl]-γ-valerolactone was contained.

Additionally, the fraction containing (R)-5-[3-(β-D-gulcopyranuronosyloxy-5-hydroxy) phenyl]-γ-valerolactone was subjected to recycling HPLC to purify. The conditions of the recycling HPLC are described below.

### <Conditions of Recycling HPLC>

·Column: Mightysil RP18 GP (20i.d.×250 mm, 5 um, manufactured by KANTOU CHEMICAL CO., INC.)
·Flow Velocity: 15 ml/min.
·Mobile Phase: acetonitrile/water/acetic acid (15/85/0.2, volume ratio (v/v/v))
·Detector: UV 270 nm

After fractionation and collection, the fraction was confirmed to contain (R)-5-[3-(β-D-gulcopyranuronosyloxy-5-hydroxy) phenyl]-γ-valerolactone by LC/MS analysis described above. The fraction containing (R)-5-[3-(β-D-gulcopyranuronosyloxy-5-hydroxy) phenyl]-γ-valerolactone was concentrated and dried by evaporator. 5 ml of water was added to the dried and solid substance to dissolve and then the solution was concentrated and dried under reduced pressure again. By repeating this operation 3 times the acid contained in the organic solvent was completely removed. Finally, 2 ml of water were added to the dried and solid substance to dissolve, and the solution was freeze-dried to obtain 28.0 mg of (R)-5-[3-(β-D-gulcopyranuronosyloxy-5-hydroxy) phenyl]-γ-valerolactone.

### [Evaluation for Compound Contained in Brain Function Improving Agent of The Present Invention]

The compound for the test: The present tests were conducted using a plurality of the compounds to confirm the effect of the compounds. The compound subjected to the tests were shown in following Table 1.

**[Table 1]**

| | |
|---|---|
| Compound A:Example1 | (R)-5-(3,5-dihydroxyphenyl)- *γ* -valerolactone |
| Compound B:Comparative example1 | (R)-5-(3,4-dihydroxyphenyl)- *γ* -valerolactone |
| Compound C:Comparative example2 | (R)-5-(3,4,5-trihydroxyphenyl)- *γ* -valerolactone |

### <Test Example 1: Analytical Research of Amount of Extracellular Acetylcholine in Mouse Brain by in vivo Microdyalysis Method>

The compound A, B, and C were administered orally to the mouse and the effect on the amount of extracellular acetylcholine in the brain was examined. The brain microdyalysis method using the mouse is proposed as a test for evaluating cognitive dysfunction including Alzheimer type dementia. The brain microdyalysis method is a system that can monitor the variation of extracellular neurotransmitter release in real time at the local parts in the brain while the test animals are moving freely without anesthesia.

### (a) Used animal, Rearing Procedure and Method for Measuring Amount of Extracellular Acetylcholine

7-weeks-old male ddY mice were purchased from Japan SLC, Inc. and subjected to the experiment after one week habituation period. The animals were reared in the rearing chamber provided with air conditioner (under the condition that the temperature was 22 °C ± 1 °C, the relative humidity was 50 ± 10 %, and the irradiation time was 12 hours (8:00 to 20:00)). The feeding method was that the commercially available MF powdery feed (manufactured by Oriental Yeast Co., ltd.) and tap water were taken freely.

In the two days before the start of the experiment, the fixing operation of the guide cannula for inserting the dialysis probe in the mouse was conducted in advance. Under the deep anesthetized conditions, the guide cannula (depth 4 mm) (manufactured by Eicom Corp.,) was inserted in and fixed on cerebral cortex prefrontal area (which was 1.9 mm forward and 0.5 mm right side from the bregma and to which the depth from cranial surface was 0.8 mm). Buprenorphine (0.01 mg/ kg manufactured by Sigma-Aldrich Japan) was administered to the mouse as analgesic after the operation. Each mouse was reared in the separate cage after the operation.

In the step of measuring extracellular acetylcholine in the brain, the dialysis probe (membrane length 3 mm) (manufactured by Eicom Corp.,) was inserted in the guide cannula and the Ringer's solution in the Japanese Pharmacopeia (containing NaCl (147.2 mM), KCl (4.0 mM), and CaCl₂ (2.2 mM); manufactured by FUSO CHEMICAL CO., LTD.) was perfused at the flow velocity of 1 µL/min. The sample obtained by the perfusion was collected every 20 minutes and was immediately continuously automatically injected in the high-speed liquid chromatography/ electrochemical detector (HPLC/ECD) system (manufactured by Eicom Corp., HTEC-500) together with the internal standard material (isopropylhomocholine, 250 fmol) and acetylcholine was measured under the condition described below.

### <Analysis Condition of Acetylcholine>

·Column: Eicompak AC-GEL (2.0 mm; i.d.×150 mm) (manufactured by Eicom Corp.,)
·Precolumn: PC-05-03 (3.0 mm; i.d.×40 mm) (manufactured by Eicom Corp.,)
·Enzyme-immobilized column: AC-Enzympak (3.0 mm; i.d.) (manufactured by Eicom Corp.,)
·Mobile Phase: 50 mM potassium hydrogen carbonate buffer containing 400 mg/L sodium decansulfonic acid and 134 µM EDTA (pH 8.3)
·Flow Velocity: 150 µl/min.
·Temperature of column: 33 °C
·Applied voltage of ECD: +450 mV
·Reference electrode: Ag/AgCl electrode
·Work electrode: platinum electrode

### (b) Administering Procedure and Measuring Procedure

The compound A prepared according to the procedure described in Manufacturing Example 1 was dissolved in 2.5 % DMSO solution in advance to prepare for the test. Two groups of 5 mice, the administration group of 30 mg/kg of the compound A and the control group were set and the test was conducted. The dialysis probe was inserted in the cerebral cortex of the mouse. After 3 hours or more had passed, it was confirmed that the amount of extracellular acetylcholine released was stabilized. The value confirmed to be stabilized was defined as basic released amount (base line). Then the compound A was orally administered at a dose of 30 mg/kg. The amount of the extracellular acetylcholine released was measured by the procedure described above in (a) Used animal and Rearing Procedure in real time for 120 minutes after the administration. The mouse in Vehicle group took only 2.5 % DMSO solution orally in the same way and the amount of the extracellular acetylcholine was measured. As a group for comparing the compounds, the tests of the compound B and the compound C were conducted in the same way.

### (c) Statistical Processing Method

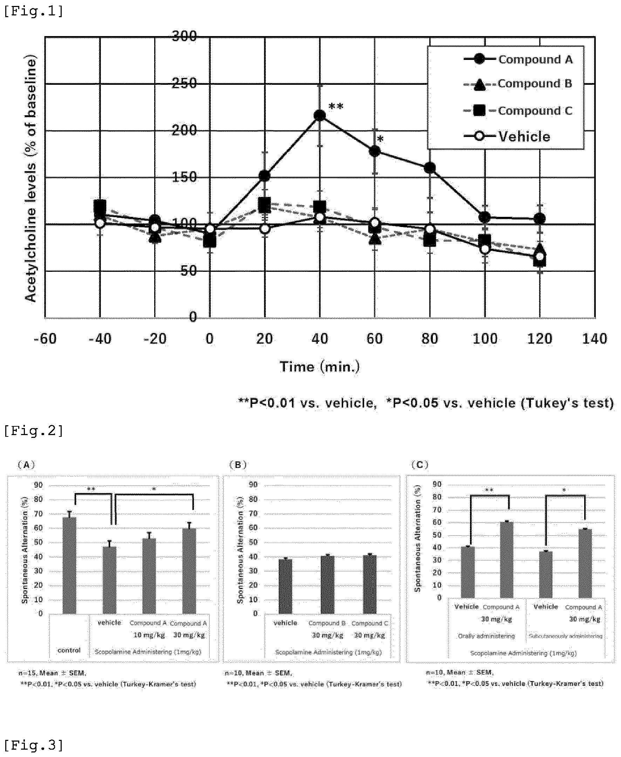

To verify the effect to the amount of extracellular acetylcholine in the brain by addition of compound administration, the comparison of test groups was conducted by ratios of the amount of extracellular acetylcholine in the brain to the base line. From the results obtained, the mean (Mean) and the standard error (SEM) were calculated. The significant difference between the compound A administration group and the Vehicle group and comparison group was assessed by Turkey-Kramer's test. The level of significance was set to * P<0.05 and **p<0.01. The results were shown in Fig.1. The vertical axis of the graph in Fig.1 represents the ratio of the amount of acetylcholine released in the brain to basic released amount.

As shown in Fig.1 the concentration of acetylcholine in the brain of the mouse was increased rapidly after the oral administration of the compound A and the amount of extracellular acetylcholine in the cerebral cortex was increased for 2 hours after the administration in comparison with the amount before the administration. Particularly the significant increase was confirmed in the compound administration group in comparison with the Vehicle group 40 and 60 minutes after the administration. On the other hand, there were not significant change of the amount of extracellular acetylcholine in the cerebral cortex in the administration groups of the compounds B and C for comparison. According to the above results it was confirmed that the compound A had the action of increasing the amount of acetylcholine which is neurotransmitter in the brain and newly that additionally the action had immediate effect.

### <Test Example 2: Action of Inhibiting Lowering of Short-term Working Memory of Scopolamine-Induced Amnesia Mouse by Y-maze Test>

The effect of inhibiting lowering of short-term memory was examined by orally or subcutaneously (intraperitoneally) administering the compound A to the mouse.

### (a) Used Animal and Rearing procedure

7-weeks-old male ddY mice were purchased from Japan SLC, Inc. and subjected to the experiment after one week habituation period. The animals were reared in the rearing chamber provided with air conditioner (under the condition that the temperature was 22 °C ± 1 °C, the relative humidity was 50 ± 10 %, and the irradiation time was 12 hours (8:00 to 20:00)). The feeding method was that the commercially available MF powdery feed (manufactured by Oriental Yeast Co., ltd.) and tap water were taken freely.

### (b) Administering Procedure and Measuring Procedure

The compound A prepared according to the procedure described in Manufacturing Example 1 was dissolved in 2.5 % DMSO solution in advance. There were 15 mice in each group, and the 30 mg/kg compound administration group and the 10 mg/kg compound administration group were established and orally administered. The mice of the control group and Vehicle group were orally administered with only 2.5% DMSO solution. In the same manner the compound B and the compound C were orally administered to 10 mice at a dose of 30 mg/kg respectively. Each mouse of the administration groups of the compound A to C and the vehicle group was intraperitoneally administered with scopolamine hydrobromide (manufactured by Sigma-Aldrich Japan S1875) (a dose of 1 mg/kg) which is muscarinic acetylcholine receptor antagonist 30 minutes after the administration. The mice of the control group which were not administered with scopolamine hydrobromide were intraperitoneally administered with (Japanese Pharmacopoeia) physiological saline solution (OTSUKA NORMAL SALINE 3311401A2026). At 30 minutes after the final administration, Y-maze test was conducted, and the working memory was evaluated. The test using the mice orally administered was separately conducted under the same condition as that described above for comparative evaluation of the intraperitoneal administration.

The Y-maze manufactured by Brain Science·idea Co., Ltd. was used for the test. The maze was the maze 3 courses of which were made into Y-letter shape having one selection point and arranged at interval of an angle of 120 degree. All of the courses had a triangular column-shape having an upper width of 12 cm, a lower width of 3 cm, a length of 40 cm, and height of 15 cm. The mouse put on the gray course (made of polyvinyl chloride) was kept in the conditions and the mouse can see nothing but the upper part. Any one of the 3 courses of the maze was set as a starting point. After placing the mouse quietly at the starting point, the number of the times the mouse entered each course was record per 8 minutes, and as well as which course and in what order the mouse entered it. The ratio of the number of three consecutive entries into different courses (the number of the alternation behavior) to the total number of the selections was calculated as Spontaneous Alternation (%) according to the following formula and was used as an index of the short-term working memory. Spontaneous Alternation (%) = the number of the alternation behavior/(the total number of the selection - 2) × 100

### (c) Statistical Processing Method

From the results obtained, the mean (Mean) and the standard error (SEM) were calculated. The significant difference between the control group and the Vehicle group and between the compound A administration group and the Vehicle group was assessed by Turkey-Kramer's test. The level of significance was set to * P<0.05 and **p<0.01. Spontaneous Alternation ratios at orally administering of compounds A to C were shown in Fig. 2 (A) and (B). The evaluation results in the kinds of methods for administering compound A were shown in Fig.2 (C).

As shown in Fig.2 (A), the decrease of spontaneous alternation due to the intraperitoneal administration of scopolamine hydrobromide which is the muscarinic acetylcholine receptor antagonist can be seen, and the disorder of the short-term memory in Vehicle group was clearly recognized. On the other hand, lowering of the short-term memory due to the administration of scopolamine was inhibited in the group in which the mice were orally administered with scopolamine after orally administered with the compound A. In particular, in the 30 mg/kg administration group, the significant effect of inhibiting the lowering of short-term working memory was confirmed, and the effect of ameliorating the impairment of the short-term working memory by administering the compound A in the living body was confirmed. On the contrary, the action of ameliorating the impairment of the short-term working memory by administering the compound B and C having a structure similar to that of the compound A was not confirmed at all. Accordingly, it was confirmed that the effect of improving the brain function was specific to the structure of the compound A.

As shown in Fig.2 (C), compound A inhibited scopolamine-induced reductions in working memory in both oral and subcutaneous administration. From the results described above, it was founded that the compound A suppressed the impairment of the short-term working memory and had the therapeutic effect on the symptom.

### <Test Example 3: Therapeutic or Recovery Effect on Novel Object Cognitive Function of Model Mouse with Psychiatric Disorder Caused by Long-term Isolated Rearing>

The action of ameliorating the lowering of the object cognitive function was examined by orally administering the compound A to the mouse reared in long-term isolation. It was proposed that the mouse reared in long-term isolation is used as a psychiatric disorder model animal exhibiting emotional disorder such as depression and anxiety, cognitive dysfunction which is the symptom of schizophrenia and the like (Neurosci Biobehav Rev. 2008; 32: 1087-1102). In this test, the mouse reared in long-term isolation under the following condition was used as the psychiatric disorder model mouse.

### (a) Used Animal and Rearing procedure

3-weeks-old male ddY mice were purchased from Japan SLC, Inc. The mice were reared in the rearing chamber provided with air conditioner (Conditions: the temperature was 22 °C ± 1 °C, the relative humidity was 50 ± 10 %, and the irradiation time was 12 hours (8:00 to 20:00)). The feeding method was that the commercially available MF powdery feed (manufactured by Oriental Yeast Co., ltd.) and tap water were taken freely.

3-weeks-old male ddY mice (n=63) were separately reared in the gray opaque cage (24×17×12 cm) in which the mice could not see the surrounding of the cage for 6 weeks. The mice were used as a psychiatric disorder model mouse exhibiting the lowering of cognitive function. The mice (n=20) collectively reared in a group of 5 mice in the transparent cage (24×17×12 cm) were used as a control group.

Four days prior to the start of the experiment, long-term isolation and group-reared mice were individually acclimated to the test observation box for 10 minutes for three consecutive days. The day before the test the mouse was allowed to freely explore two objects of different shape and color (acquisition trial) and was trained to recognize the object for 10 minutes.

### (b) Administering Procedure and Measuring Procedure

The compound A was dissolved in in 2.5 % DMSO solution in advance. The mice reared in long-term isolation were divided into 3 groups of 21 mice and the administration groups of the compound (10 mg/kg or 30 mg/kg) and Vehicle group were set. The mice reared in a group for comparison and administered with2.5 % DMSO solution were used for the test as a control group.

Just after the acquisition trial, the mice in 4 groups which were separated into Vehicle group (long-term isolated rearing, n=20, the oral administration of 2.5 % DMSO solution), the administration group of the compound A (long-term isolated rearing, n=20, the oral administration of the compound A (30 mg/kg)), the administration group of the compound A (long-term isolated rearing, n=20, the oral administration of the compound A (10 mg/kg)) and the control group (rearing in a group, n=20, the oral administration of 2.5 % DMSO solution) were orally administered.

24 hours after the oral administration, the evaluation test for novel object cognitive function was carried out. One object of two objects put in the observation box was replaced with the novel object and the time how long the mouse explored the novel object was measured in the test time of 5 minutes. The object cognitive function of the mouse was evaluated by Discrimination index (%) calculated using the following formula. Discrimination index (%) = (the exploratory time of the novel object-the exploratory time of the familiar object)/(the exploratory time of the novel object + the exploratory time of the familiar object) × 100

### (c) Statistical Processing Method

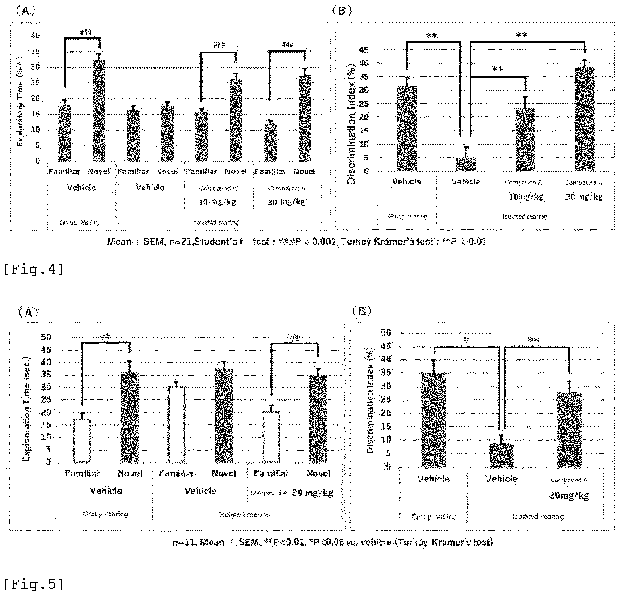

From the results obtained, the mean (Mean) and the standard error (SEM) were calculated. The significant difference was assessed between the administration group and the control group by using Student's t-test as a method of the statistical processing of the exploratory time and by using Turkey-Kramer's test as a method of the statistical processing of Discrimination index. The level of significance was set to ### P<0.001 and **p<0.01, respectively. The results of the exploratory time (Exploratory time) and Discrimination index were shown in Fig.3(A) and (B). Note that "Familiar" and "Novel" in Fig.3(A) were meant to be the familiar object and the novel object, respectively.

As shown in Fig.3 (A), the exploratory time of the novel object of the mice reared in long-term isolation and orally administered with the compound A in the administration groups of both 10 mg/kg and 30 mg/kg significantly increased compared with the Vehicle group. As shown in Fig.3(B), in Discrimination index evaluation, the score equal to the score obtained in the group of the mice reared in a group which is the control group was obtained in the administration group of 30 mg/kg of the compound A. This confirmed that cognitive dysfunction due to psychiatric stress caused by the long-term isolated rearing was significantly ameliorated by the oral administration of the compound A and that there was the remarkable therapeutic effect.

The results described above was confirmed that the oral administration of the compound A significantly ameliorated the symptoms of psychiatric disorder induced by the long-term stress isolated rearing and exhibited the therapeutic effect.

### <Test Example 4: Effect of Inhibiting Onset of Cognitive Dysfunction by Long-term Administration of Compound A to Mouse Reared in Long-term Isolation>

The compound A was orally administered for a long time and the effect of preventing the onset of cognitive dysfunction caused by the long-term isolated rearing was examined.

### (a) Used Animal and Rearing procedure

3-weeks-old male ddY mice were purchased from Japan SLC, Inc. The mice were reared in the rearing chamber provided with air conditioner (under the condition that the temperature was 22 °C ± 1 °C, the relative humidity was 50 ± 10 %, and the irradiation time was 12 hours (8:00 to 20:00)). The feeding method was that the commercially available MF powdery feed (manufactured by Oriental Yeast Co., ltd.) and tap water were taken freely.

To prevent the moisture absorption of the compound A during the long-term administration, the compound A and the excipient (dextrin manufactured by Showa Sangyo Co., Ltd. commercial name "J-SPD") were mixed homogeneously in advance to prepare the powder containing 10 % the compound A.

### (b) Administering Procedure and Measuring Procedure

70 mg of the excipient containing 10 % the compound A and 100g of the MF powdery feed were mixed homogeneously to prepare the feed mixed with the compound A. As a control feed, the MF powdery feed only containing 70 mg of the excipient without the compound A was used. 3-weeks-old male ddY mice (n=22) were separately reared in the gray opaque cage (24×17×12 cm) in which the mice could not see the surrounding of the cage. Among them, 11 mice were fed on the MF powdery feed containing the compound A and 11 mice of Vehicle group were fed on the MF powdery feed without the compound. These mice were continuously reared in isolation for 5 weeks, and during that period, the former mice were continuously fed on the MF powdery feed containing the compound A.

The mice (n=11) in the control group were reared in a group in the transparent cage (24×17×12 cm) and fed on the MF powdery feed without the compound A.

After the long-term rearing of 5 weeks, the object cognitive function was evaluated by the same method as that described in Test Example 3 and the effect of preventing the onset of cognitive dysfunction by administering the compound A during the long-term rearing was examined.

### (c) Statistical Processing Method

From the results obtained, the mean (Mean) and the standard error (SEM) were calculated. The significant difference of exploratory time in each group was assessed by using Student's t-test and the significant differences between the control group and Vehicle group and between the administration group of compound A and Vehicle group of Discrimination index were assessed by using Turkey-Kramer's test. The level of significance of Student's t-test was set to # P<0.05 and ##P<0.01 and the level of significance of Turkey-Kramer's test was set to *P<0.05 and **p<0.05. The results of the exploratory time and Discrimination index were shown in Fig.4(A) and (B).

As shown in Fig.4(A) and (B), the onset of cognitive dysfunction caused by the long-term isolated rearing stress was significantly inhibited in the group in which the mice took the compound A for a long time. It has been proposed that the model mouse reared in long-term social isolation has been used as a model animal for psychiatric disorder exhibiting emotional disorder such as depression and anxiety, cognitive dysfunction exhibiting the symptom of schizophrenia, and the like. Above results indicated the possibility that taking the compound A for a long time prevented the onset of psychiatric disorder such as depression and schizophrenia caused when stress load.

### <Test Example 5: Evaluation for Alleviation of Depressive Symptoms Derived from Inflammation in Brain Caused by Administration of LPS>

In regard to the depressive symptoms induced by taking LPS (Lipopolysaccharide), the effect of inhibiting the symptom by oral administration of the compound A was examined. The inflammation in the brain is thought to be one of the main onset factors of depression. It is known that LPS causes the inflammation in the brain by inducing the decrease in brain-derived neurotrophic factor and the variation of the density of dendrite of neuron, activating microglia and the like and induce depressive symptoms. Thus, in this test, the antidepressant action by the oral administration of compound A was examined using the model mouse with the inflammation in brain induced by administering LPS intraperitoneally.

### (a) Used Animal and Rearing procedure

7-weeks-old male C57/BL6J mice (purchased from Japan SLC, Inc.) were used. The mice were reared in the rearing chamber provided with air conditioner (Conditions: the temperature was 22 °C ± 1 °C, the relative humidity was 50 ± 10 % , and the irradiation time was 12 hours (8:00 to 20:00)). The feeding method was that the commercially available MF powdery feed (manufactured by Oriental Yeast Co., ltd.) and tap water were taken freely. After 1 week acclimation, 8-weeks-old mice were used for the test.

### (b) Administering Procedure and Measuring Procedure

The compound A was dissolved in in 2.5 % DMSO solution in advance. The compound A was orally administered to the mice at a dose of 30 mg/kg and 10 mg/kg. The mice in the control group and Vehicle group were orally administered with only 2.5 % DMSO solution. Each group was set to have 15 mice.

To induce the inflammation in the brain after 30 minutes, LPS (serotype O111: B4) were intraperitoneally administered to the mice in the administration groups of the compound A and Vehicle group at a dose of 0.5 mg/kg. The control mice, which did not develop inflammation in the brain, received intraperitoneal saline in the same manner.

In 24 hours after the intraperitoneal administration of LPS, forced swimming test was conducted. The depressive symptoms were evaluated by measurement of the immobility time.

Water (25 ± 1 °C) was put into the acryl resin cylinder (diameter 19 cm, height 25 cm) up to a height of 13 cm. The subject mouse was forced to swim for 6 minutes one by one and the state was video-recorded. After the test, the mouse was quickly pulled up from the water and wiped with the paper towel. "The state that the mouse floated on the water surface without moving its limbs and the like" was regarded as immobility and the Immobility Time (sec.) was measured during the test of 6 minutes.

### (c) Statistical Processing Method

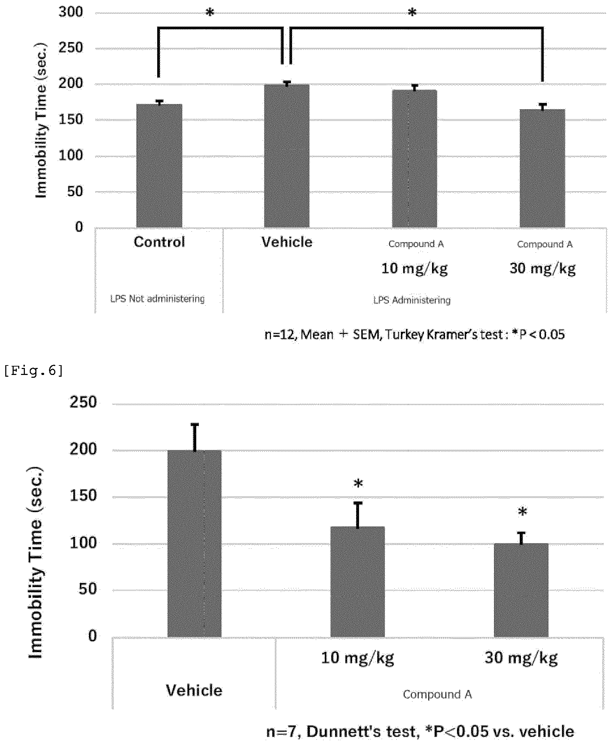

From the results obtained, the mean (Mean) and the standard error (SEM) were calculated. The significant difference was assessed between the control group and Vehicle group and between the compound A administration group and the control group by using Turkey-Kramer's test. The level of significance was set to *p< 0.05. The results were shown in Fig.5. The vertical axis of the graph in Fig.5 represents the immobility time during the test. The immobility time was significantly increased in the Vehicle group having the mice administered with LPS compared to the control group. The increase of the immobility time was reduced in the administration groups of the compound A and it was observed that the immobility time in the administration group of 30 mg/kg was on the same level as that in the control group. The model mouse with the inflammation in brain induced by administering LPS intraperitoneally is used as a depression model animal. These results showed that the compound A significantly alleviated the depressive symptoms due to the inflammation in the brain and had the effect of preventing the onset.

### <Test Example 6: Evaluation for Ameliorating Action to the Depressive Symptoms Caused by Long-term Isolated Rearing>

The psychiatric disorder model mouse which was reared in isolation for 5 weeks was orally administered with the compound A and 30 minutes after the administration subjected to the Forced swimming test. The effect of improving depressive symptoms was examined.

### (a) Used Animal and Procedure

The 3-weeks old male ddY mouse which was reared in isolation for 5 weeks in the same manner as in Test Example 3 was used for the test.

### (b) Administering Procedure and Measuring Procedure

The compound A was dissolved in in 2.5 % DMSO solution in advance. After long-term isolated rearing the mice were divided into 3 groups of 7 mice and the administration groups of the compound A (the administration group of 30 mg/kg, the administration group of 10 mg/kg) and Vehicle group (the administration group of 2.5 % DMSO solution) were set and the oral administration was carried out.

In 30 minutes after the oral administration, the forced swimming test was carried out in the same manner as in Test Example 5. The immobility time was measured during the test of 6 minutes and the depressive symptoms of the mouse was evaluated.

### (c) Statistical Processing Method

From the results obtained, the mean (Mean) and the standard error (SEM) were calculated. The significant difference was assessed between the compound A administration group and the Vehicle group by using Dunnett's test. The level of significance was set to *P< 0.05. The results were shown in Fig.6. The vertical axis of the graph in Fig.6 represents the immobility time during the test.

As shown in Fig.6, the immobility time was significantly reduced in both the 10mg/kg and 30mg/kg groups in the long-term isolated mice treated orally with compound A compared with the vehicle group, and the antidepressant effect on depressive symptoms was confirmed. It has been proposed that the model mouse reared in long-term social isolation has been used as a model animal for psychiatric disorder exhibiting emotional disorder such as depression and anxiety, cognitive dysfunction exhibiting the symptom of schizophrenia, and the like. The results of the present invention confirmed that the depressive symptoms were significantly ameliorated by orally administering with the compound A and that there was the remarkable therapeutic effect on depression in the model mouse with psychiatric disorder induced.

### Industrial Applicability

The present invention can provide the brain function improving agent for preventing the onset of, ameliorating or treating neurodegenerative diseases such as dementia, central nervous system diseases caused by psychiatric disorders such as depression and schizophrenia, and cognitive dysfunction and depressive symptoms caused by central nervous system diseases, and containing the specific compound reported as the catechin metabolites and the derivatives thereof. The present invention provides the supplement, the food and beverage, and medicine having the food-derived ingredient as the active ingredient, which are safe and can be taken daily for self-medication.

## Claims

1. A brain function improving agent for preventing onset of, treating, and/or ameliorating central nervous diseases, **characterized by** containing, as an active ingredient, at least one of 5-(3,5-dihydroxyphenyl)-γ-valerolactone represented by a following formula (I): 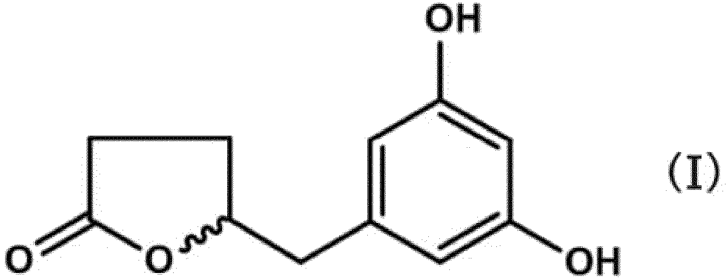
(In the formula, wavy line represents that the steric configuration is R-configuration or S-configuration.),
a salt of said compound, and a conjugate of said compound.

2. The brain function improving agent according to Claim 1, wherein the central nervous diseases are neurodegenerative diseases or psychiatric disorder.

3. The brain function improving agent according to Claim 1 or 2, wherein central nervous diseases are diseases accompanied by cognitive dysfunction.

4. The brain function improving agent according to any one of Claims 1 to 3 that activates muscarinic receptors by enhancing acetylcholine release and increasing extracellular acetylcholine in the brain.

5. A composition for preventing onset of, treating, and/or ameliorating central nervous diseases, containing the brain function improving agent according to any one of Claims 1 to 4.

6. A supplement for improving brain function containing the composition according to Claim 5.

7. Food and drink for improving brain function containing the composition according to Claim 5.

8. Medicine or quasi-drugs for improving brain function containing the composition according to Claim 5.

9. An agent for enhancing acetylcholine release or increasing extracellular acetylcholine in the brain **characterized by** containing, as an active ingredient, at least one of 5-(3,5-dihydroxyphenyl)-γ-valerolactone represented by the following formula (I): 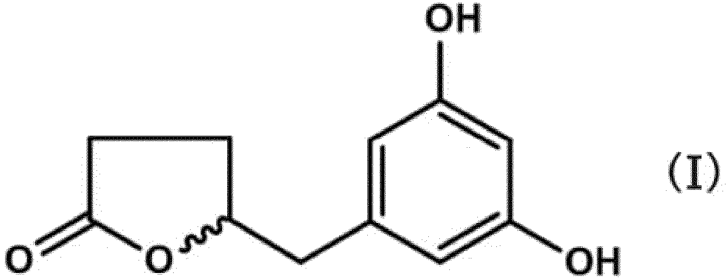
(In the formula, wavy line represents that the steric configuration is R-configuration or S-configuration.),
a salt of said compound, and a conjugate of said compound.
